**Europäisches Patentamt**

**European Patent Office**

**Office européen des brevets**

(11) Publication number: **0 432 419 B1**

# EUROPEAN PATENT SPECIFICATION

(45) Date of publication of patent specification: **19.10.94** (51) Int. Cl.⁵: **C07K 3/08**, C07K 13/00

(21) Application number: **90120309.1**

(22) Date of filing: **23.10.90**

(54) **Method for solubilization and naturation of somatotropins utilizing low urea concentration.**

(30) Priority: **05.12.89 US 446280**

(43) Date of publication of application:
**19.06.91 Bulletin 91/25**

(45) Publication of the grant of the patent:
**19.10.94 Bulletin 94/42**

(84) Designated Contracting States:
**AT BE CH DE DK ES FR GB GR IT LI LU NL SE**

(56) References cited:
**EP-A- 0 173 215**
**EP-A- 0 279 619**
**EP-A- 0 336 324**

(73) Proprietor: **AMERICAN CYANAMID COMPANY**
**One Cyanamid Plaza**
**Wayne, NJ 07470-8426 (US)**

(72) Inventor: **McCoy, Kevin Michael**
**921 Garden Street**
**Hoboken, New Jersey 07030 (US)**

(74) Representative: **Wächtershäuser, Günter, Prof. Dr.**
**Patentanwalt**
**Tal 29**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

The present invention relates to the use of a low molar aqueous concentration of urea in combination with an aqueous alkaline solution as a solubilization and naturation method for proteins. More importantly, with the advent in recent years of recombinant DNA technology responsible for production of many proteins and polypeptides, methods for effectively and efficiently solubilizing such proteins during the refining process are key to providing an economic large-scale production of those products.

Many methods for protein solubilization and naturation have been studied. For example, in US patent 4511503, refractile bodies insoluble as granules of aggregated denatured somatotropin are found in the cytoplasm of the whole microorganism, E. Coli, in which they are produced. These refractile bodies are formed by overproduction of the specified protein of interest, such as somatotropin, as a result of genetically engineering the E. Coli cell to purposely overproduce that desired protein. Oftentimes, the only mechanism by which to treat the refractile bodies is with a strong denaturant or chaotropic agent in order to cause the improperly folded molecules of both the desired product (protein) and the E. Coli proteins to unfold and become soluble. In addition to causing this denaturation, proteins must then "renature" in the proper monomeric form in order to be biologically active. This monomeric form is especially important for somatotropin. Oftentimes, in order to obtain the active product protein, strong denaturants such as guanidine hydrochloride or urea at very high concentrations have been used.

In addition to these strong denaturant methods, certain chaotropic agents such as sodium dodecylsulfate (SDS) as disclosed in US Patent 4677196 or weak denaturants such as urea preferably used at molar concentrations of 3 to 5 molar such as disclosed in the US Patent 4731440 or no urea as disclosed in US Patent Application 2854775 December 16, 1988 have been used. Figure 1 compares all three urea processes.

Each of the methods have certain associated problems with them. Guanidine hydrochloride is expensive and must be replaced during the naturation process in order for naturation to occur. SDS is an effective denaturant and less expensive than guanidine hydrochloride, but it binds to the denatured protein so tightly that it makes complete removal from the protein difficult and increases processing costs. Urea is usually used as a weaker denaturant or chaotropic agent, but even methods utilizing urea have problems associated with it, such as contamination of final products, handling, storage and waste treatment problems. Therefore, any method which uses low concentrations of urea, an agent that helps to enhance recovery of the proper monomeric form of the product protein, is specifically needed in the art.

Additionally, it is the obtaining of this monomer product that is important in arriving at product proteins which have the physiological and biological activities of somatotropins. The somatotropin monomer consists of approximately 191 amino acid residues and has molecular weight of roughly 22,000 daltons. The monomer is not linked or noncovalently bonded to other similar monomer molecules.

In addition to the monomer form, somatotropins can exist in dimer form which means that two monomer molecules are covalently linked through intermolecular disulfide bonds or noncovalently associated with one another. The dimer protein molecule consists of double the number of amino acids and double the molecular weight of monomer and is formed, unfortunately, by the inefficiencies of processes used to solubilize and renature the refractile bodies. In other words, it is formed by the isolation, naturation and purification process required to obtain active product protein from inactive inclusion (refractile) bodies combined within the microorganism cell.

The present invention provides a commercially feasible process for solubilizing proteins by effectively combining the use of urea as a solubilizing agent with optimal naturation conditions used in aqueous dissolution procedures utilizing no urea. Naturation includes oxidation of the cystine residues to form cysteine bonds. The use of very low molar concentrations of urea results in yield advantages as compared to other urea processes.

SUMMARY OF THE INVENTION

The present invention relates to the process for solubilization and naturation of somatotropins by using low urea concentrations in combination with an aqueous alkaline dissolution process in order to maximize monomer formation in the refractile body solubilization step, as part of an overall process for the production of purified somatotropin (recombinant). This method comprises dispersing somatotropin refractile bodies in a low urea molar concentration, of 1.8 to 2.2 molar urea, for sufficient time to solubilize said refractile bodies (2 to 20 minutes), at a pH necessary to dissolve said refractile bodies and then diluting said solution at least three fold with water so that the resulting urea solution is a very weak, low urea concentration; i.e. 0.4 molar. The resulting solution's pH is maintained at a pH to allow proper monomeric folding and oxidation of the somatotropin and for sufficient time for said monomeric folding and oxidation to occur.

It is an object of the present invention, therefore, to provide a process for solubilizing refractile bodies, especially those of somatotropin, by combining an aqueous alkaline dissolution process with a low molar urea concentration. This results in not only beneficial and preferred monomer formation but has yield advantages over both the higher urea concentration process disclosed in the art and no urea usage. Further, an object of the present invention is to minimize the formation of dimers and yet allow for proper monomer naturation. These and other objects of the invention will become apparent by the more detailed description of the invention provided herein below.

## BRIEF DESCRIPTION OF THE FIGURES

Figure 1 schematically provides a comparison of a higher molar concentration urea process, a non urea process and the process of the present invention. "Inclusion bodies" is an analogous term for "refractile bodies" and may be used interchangeably herein.

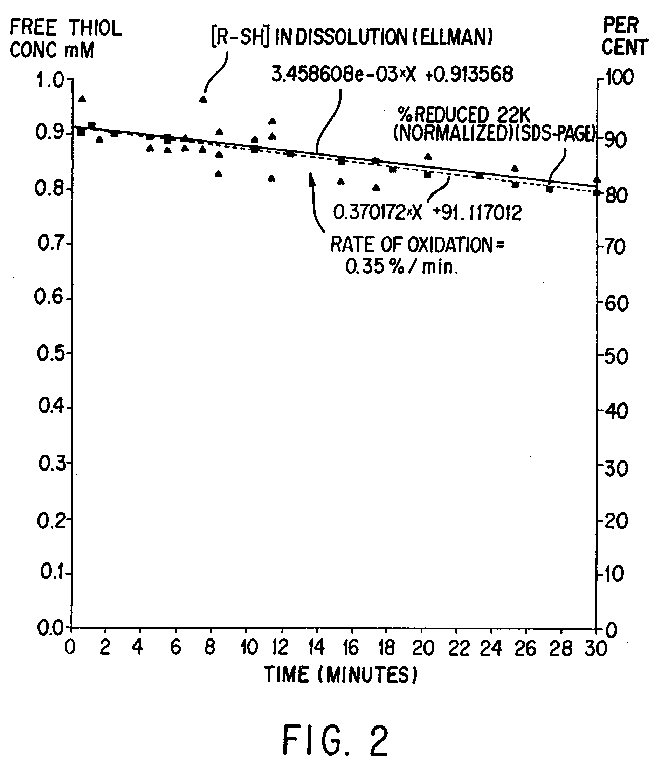

Figure 2 indicates that protein oxidation as measured by the Ellman reaction or by nonreducing SDS-PAGE in the first twenty minutes of the present process takes place at a rate of about 2% to 7% totally, thereby indicating the majority of oxidation is taking place in the dilute urea condition in comparison to the oxidation claimed in US Patent 4652630.

Figure 3 is an indication that at the 5 minute holding of the refractile bodies at one of the highest urea concentrations, i.e. 2 molar urea, no significant oxidation occurs.

## DETAILED DESCRIPTION OF THE INVENTION

The present invention relates to a method for solubilization and naturation of somatotropins comprising dispersing somatotropin refractile bodies into water containing about 1.8 to 2.2M urea; adjusting the pH of the refractile bodies in water to a level to effect solubilization; such that somatotropin protein concentration is about 2 to 10 g/L; holding said resulting solution at that pH and at that concentration for about 2 to 20 minutes; diluting with water; and adjusting the pH to 11.0 to 12.5, with a resulting urea concentration of about 0.4 molar and a somatotropin content of about 0.4 to 2.0 g/L; maintaining the solution at the readjusted pH for time sufficient to result in the somatotropin content in solution to be composed of properly folded monomeric somatotropin in good yield (approximately 5 to 10 hours).

"Somatotropin" as used herein denotes (1) animal growth hormone, derivatives, analogs and fragments of whatever species, for example, human, bovine, porcine, ovine, caprine, equine, avian (chicken, duck, goose, turkey), fish and others; (2) precursors to growth hormone, such as reduced (-SH) growth hormone and S-protected growth hormone, for example, growth hormone S-sulfonate; (3) variants of growth hormone or its precursors, for example, structures which have been modified to lengthen and/or shorten the growth hormone amino acid sequence, for example the 20K variant of human growth hormone, methionyl human growth hormone, delta 7 and delta 9 porcine growth hormone and the like; and (4) analogs of growth hormone or its precursors, for example structures in which the growth hormone amino acid sequence has been modified by deletion or replacement of one or more amino acid residues. Both recombinantly derived somatotropin and naturally occurring somatotropin, as well as any other type of somatotropin, may be utilized in accordance with the present invention.

The first step in the novel method is the dispersal of the somatotropin refractile bodies into a low urea molar concentration of water (preferably deionized water) at a suitable concentration. Concentration of somatotropin is an important factor of the present invention and must be monitored to fall between 2 to 10 g/L. A suitable concentration of urea is about 1.8 to 2.2M and preferably about 2M.

The pH of the concentrated somatotropin is adjusted to a range from about pH 11.5 to about pH 12.5, preferably about pH 12.0 to about pH 12.2 to solubilize the somatotropin in an aqueous urea solution. Any strong base may be used to adjust the pH of the solution (e.g. the addition of sodium hydroxide or potassium hydroxide). This generally takes place in a relatively short period of time, about two to twenty minutes is typical. Then, this solution is diluted with water, preferably deionized water, at least three-fold, preferably five-fold (three to five-fold). Somatotropin concentration is now about 2 g/L to 10 g/L, with 4 to 6 g/L preferred.

After the somatotropin refractile bodies are dissolved, the pH may be readjusted to a range from about pH 11 to about pH 12.5. Lowering the pH increases the rate of naturation. The pH can be lowered by any suitable method, for example by the addition of phosphoric acid. The readjusted range is preferably about pH 11.3 to pH 12.0 for minimization of dimer. Especially preferred is a pH of 11.5 to 11.7.

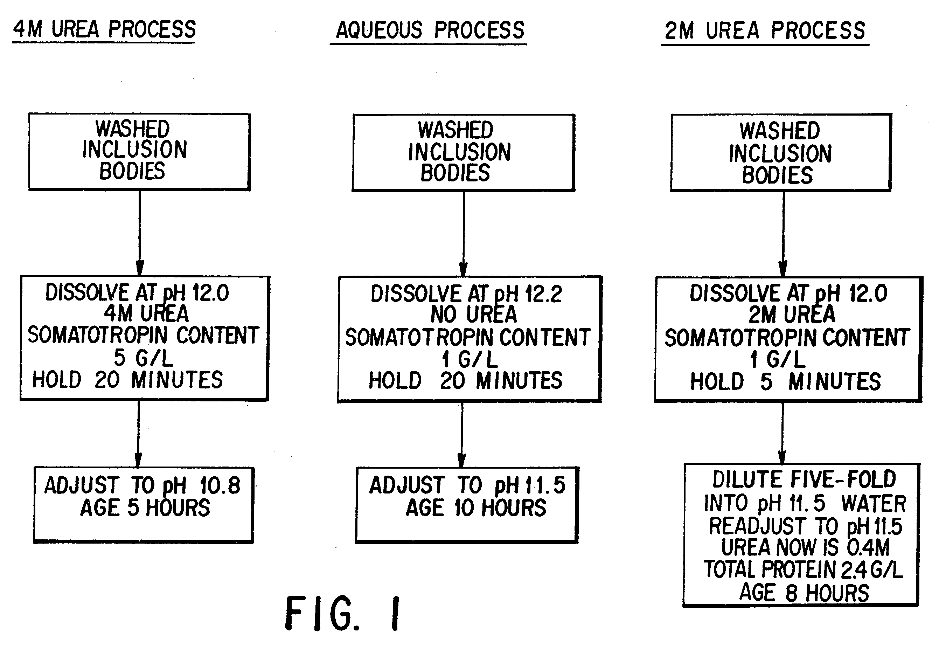

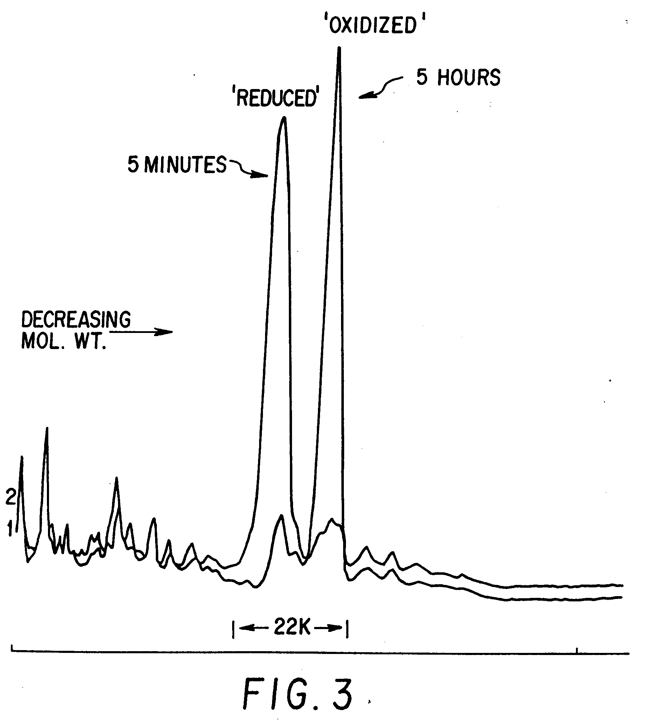

Figure 1 compares the present process with a no urea process (aqueous process) and 4M urea process. Further, direct comparison of a representative higher urea process, as claimed in U.S. patent 4652630, with the present process is made and indicates that contrary to what is stated in that patent, the present system does not result in rapid oxidation. In fact, the oxidation rate of the present process is calculated to be about 0.35% per minute or 2% to 7% for the two to twenty minute low urea exposure. Also, Figure 3 confirms that the degree of oxidation taking place in the low urea dissolution period is insignificant considering the end purpose of requiring 100% oxidized monomer which is biologically active. These comparisons provide evidence of the unique characteristics of the present process, wherein good yields of end product, in spite of the teaching of the references, are obtained.

Additionally, a reducing agent such as beta-mercaptoethanol or dithiothreitol may be added to the solution. If used, this reducing agent should be added just before the three to five-fold dilution step rather than prior to solubilization.

Four different laboratory lots of somatotropin are exposed to the process of the present invention and the results are provided in Table I.

## TABLE I

### Comparison of Monomer Yields from 2M Urea, 4M Urea and Aqueous Processes using Different Lots of Washed Refractile Bodies

| Lot ID | Monomer Yield, %* | | |
| --- | --- | --- | --- |
| | 2M Urea | 4M Urea | Aqueous |
| A | 88.5 (100) | 80.3 (90.7) | 73.3 (82.8) |
| B | 75.1 (100) | 67.0 (89.2) | 59.6 (79.4) |
| C | 70.2 (100) | 58.3 (83.0) | 55.8 (79.5) |
| D | 70.7 (100) | 59.1 (83.6) | 62.7 (88.7) |

*-Monomer yield is calculated as the ratio of 22K dalton somatotropin concentration as measured by gel permeation chromatography to total somatotropin concentration as measured by rp-HPLC.

-Percent monomer normalized to 2M urea process is in parenthesis.

4

EXAMPLE 1

BOVINE SOMATOTROPIN

Fermentation mash containing E. Coli cells which have been genetically modified to produce bovine somatotropin refractile bodies (also called inclusion bodies) is centrifuged to separate the cells from the broth. The cells are reslurried and disrupted using two passes at 8000 psig through a Gaulin homogenizer. The suspension is centrifuged and the pellet reslurried and treated with lysozyme and Triton\ x-100 detergent at 37°C. The suspension is centrifuged and the pellet is washed twice with water and is centrifuged after each wash.

The resulting pellet, containing the insoluble denatured bovine somatotropin (bST), is dispersed into deionized water at 25°C. The volume of water is chosen such that after urea is added, somatotropin concentration is 5 g/L. Solid urea is added until the urea concentration is 2M. The resultant slurry is heated to 25°C to negate the urea endotherm. The pH is then adjusted to 12.0 by addition of IN sodium hydroxide resulting in visible dissolution of the inclusion bodies. After twenty minutes, the solution is then diluted into four volumes of pH 11.5 deionized water and aged for seven hours. After dilution into four volumes of pH 11.5 water, the somatotropin concentration is 1 g/L.

At the end of aging the pH of the solution is lowered to 10.8 using 1M phosphoric acid. The solution is ultrafiltered and diafiltered on 100K dalton cut-off hollow-fiber cartridge such as an Amicon\ H26P100-43. The permeate is then concentrated using a 5K dalton cut-off spiral-wound cartridge ultrafilter such as Koch\ K-131A.

The concentrated solution is adjusted to pH 9 using 1M phosphoric acid and applied at 15g bST per L resin to an anion-exchanger such as DEAE-Sepharose Fast Flow\ which had been equilibrated with 10mM borate, pH 9. After washing with the equilibration buffer, the bST is eluted using a 100mM NaCl, 10mM borate solution, at pH 9. The bST peak is concentrated and desalted with an ammonia solution using an ultrafilter with 10K dalton cut-off cassettes such as Millipore Pellicon\until the conductivity of the permeate is less than 300 microsiemens/cm. The desalted solution at approximately 100 g/L is lyophilized to yield bovine somatotropin (bST) which passes established biological and chemical tests. The yield of lyophilized bovine somatotropin is 48% from the fermentation broth.

EXAMPLE 2

PORCINE SOMATOTROPIN

Fermentation mash containing E. Coli cells which have been genetically modified to produce porcine somatotropin (pST) inclusion bodies is centrifuged to separate the cells from the broth.

The resulting pellet, containing the insoluble denatured porcine somatotropin (pST), is dispersed into deionized water at 25°C. The volume of water is chosen such that after urea is added the somatotropin concentration will be 5 g/L. Solid urea is added until the urea concentration is 2M. The slurry is heated to 25°C to negate the urea endotherm as in Example 1. The pH is then adjusted to 12.0 by addition of IN sodium hydroxide resulting in visible dissolution of the inclusion bodies. After twenty minutes the solution is then diluted into four volumes of pH 11.5 deionized water and aged for seven hours. At the end of aging the pH of the solution is lowered to 10.8 using 1M phosphoric acid. After dilution into four volumes of pH 11.5 water, the somatotropin concentration is 1 g/L. The solution is ultrafiltered and diafiltered on an Amicon\ H26P100-43 100K dalton cut-off hollow-fiber cartridge. The permeate is then concentrated using a Koch\ K-131A 5K dalton cut-off spiral-wound cartridge ultrafilter.

The concentrated solution is adjusted to pH 9 using 1M phosphoric acid and applied at 15g pST per L resin to a DEAE-Sepharose Fast Flow\ anion-exchanger which had been equilibrated with 10mM borate, pH 9. After washing with the equilibration buffer, the pST is eluted using a 100mM NaCl, 10mM borate solution, at pH 9. The pST peak is concentrated and desalted with a dilute ammonia solution using a Millipore Pellicon\ultrafilter with 10K dalton cut-off cassettes until the conductivity of the permeate is less than 300 microsiemens/cm. The desalted solution at approximately 100 g/L is lyophilized to yield porcine somatotropin which passes established biological and chemical tests.

**Claims**

1. A process for solubilization and naturation of somatotropins, said process comprising: dispersing somatotropin refractile bodies in an aqueous solution of 1.8 to 2.2M urea at a pH and for sufficient time to solubilize said refractile bodies and then diluting said solution at least three-fold with water at a pH and for a time sufficient to result in properly folded monomeric somatotropin.

2. A process according to Claim 1, wherein said time to solubilize said refractile bodies is about 2 to 20 minutes; wherein said pH is 11.0 to 12.5; wherein said time sufficient to result in properly folded monomeric somatotropin is 5 to 10 hours; and wherein said somatotropin is human, bovine, porcine, ovine, equine, caprine, fish or avian somatotropin.

3. A process according to Claim 2, additionally comprising: adding to the urea solution, prior to the dilution with at least three-fold water, a reducing agent.

4. A process according to Claim 3, wherein said reducing agent is beta-mercaptoethanol, dithiothreitol or combinations thereof.

5. A process according to Claim 4, wherein said somatotropin is human, bovine, porcine, ovine, equine, caprine, fish or avian somatotropin.

6. A process for the isolation and purification of recombinantly-produced somatotropin, said process comprising: washing refractile bodies containing said somatotropin and other proteins with water; dissolving said refractile bodies at pH 11.0 to 12.5 in about 1.8 to 2.2M urea such that the somatotropin concentration is about 2 to 10 g/L; holding said solution at said pH for about two to twenty minutes; diluting said solution at least three-fold with water; adjusting the pH of said diluted solution to 11.3 to 12.0, such that the urea concentration is about 0.4 molar and the somatotropin concentration is about 0.4 to 2.0 g/L; and holding said solution for 5 to 10 hours.

7. A process according to Claim 6, wherein said somatotropin is human, bovine, porcine, ovine, equine, caprine, fish or avian somatotropin.

8. A process according to Claim 7, wherein said time is 7 to 8 hours.

**Patentansprüche**

1. Verfahren zum Auflösen und Naturieren von Somatotropinen, umfassend:
Dispergieren refraktiver Somatotropin-Körper in einer wässerigen Lösung von 1,8 bis 2,2 M Harnstoff bei einem pH und für eine genügende Zeit, um die refraktiven Körper aufzulösen und
Verdünnen der Lösung um mindestens das Dreifache mit Wasser bei einem pH und für eine Zeit, die genügen, um richtig gefaltetes monomeres Somatotropin zu ergeben.

2. Verfahren nach Anspruch 1, worin die Zeit zum Auflösen der refraktiven Köper etwa 2 bis 20 Minuten berägt, der pH 11,0 bis 12,5 ist, die zur Bildung richtig gefalteten monomeren Somatotropins genügende Zeit 5 bis 10 Stunden beträgt und worin das Somatotropin menschliches, Rinder-, Schweine-, Schafs-, Pferde-, Ziegen-, Fisch- oder Vogel-Somatotropin ist.

3. Verfahren nach Anspruch 2, das zusätzlich die Zugabe eines Reduktionsmittels zu der Harnstofflösung vor der Verdünnung mit der mindestens dreifachen Wassermenge umfaßt.

4. Verfahren nach Anspruch 3, worin das Reduktionsmittel $\beta$-Mercaptoethanol, Dithiotreit oder deren Kombinationen ist.

5. Verfahren nach Anspruch 4, worin das Somatotropin, menschliches, Rinder-, Schweine-, Schafs-, Pferde-, Ziegen-, Fisch- oder Vogel-Somatotropin ist.

6. Verfahren zum Isolieren und Reinigen rekombinant erzeugten Somatotropins, umfassend:
Waschen der Somatotropin und andere Proteine enthaltenden refraktiven Körper mit Wasser,
Auflösen der refraktiven Körper bei pH 11,0 bis 12,5 in etwa 1,8 bis 2,2 M Harnstoff derart, daß die Somatotropin-Konzentration etwa 2 bis 10 g/l beträgt, Halten der Lösung bei dem genannten pH für etwa 2 bis 20 Minuten,
Verdünnen der Lösung um das mindestens Dreifache mit Wasser,

Einstellen des pH der verdünnten Lösung auf 11,3 bis 12,0 derart, daß die Harnstoff-Konzentration etwa 0,4-molar ist und die Somatotropin-Konzentration etwa 0,4 bis 2,0 g/l beträgt und

Halten dieser Lösung für 5 bis 10 Stunden.

7. Verfahren nach Anspruch 6, worin das Somatotropin menschliches, Rinder-, Schweine-, Schafs-, Pferde-, Ziegen-, Fisch- oder Vogel-Somatotropin ist.

8. Verfahren nach Anspruch 7, worin die Zeit 7 bis 8 Stunden beträgt.

**Revendications**

1. Procédé pour la solubilisation et la naturation de somatotropines, ledit procédé comprenant : la dispersion de corps réfringents de somatotropine dans une solution aqueuse d'urée 1,8 à 2,2 M à un pH et pendant un temps suffisant pour solubiliser lesdits corps réfringents, puis la dilution de ladite solution d'un facteur d'au moins 3 avec de l'eau, à un pH et pendant un temps suffisant pour obtenir de la somatotropine monomère convenablement repliée.

2. Procédé selon la revendication 1, dans lequel ledit temps de solubilisation desdits corps réfringents est d'environ 2 à 20 minutes ; ledit pH est de 11,0 à 12,5 ; ledit temps suffisant pour obtenir de la somatotropine monomère convenablement repliée est de 5 à 10 heures ; et ladite somatotropine est de la somatotropine humaine, bovine, porcine, ovine, équine, caprine, aviaire ou de poisson.

3. Procédé selon la revendication 2 comprenant de plus : l'addition d'un agent réducteur à la solution d'urée avant la dilution d'un facteur d'au moins 3 avec l'eau.

4. Procédé selon la revendication 3, dans lequel ledit agent réducteur est le $\beta$-mercaptoéthanol, le dithiothréitol ou une de leurs combinaisons.

5. Procédé selon la revendication 4, dans lequel ladite somatotropine est de la somatotropine humaine, bovine, porcine, ovine, équine, caprine, aviaire ou de poisson.

6. Procédé pour l'isolement et la purification de somatotropine produite par recombinaison, ledit procédé comprenant : le lavage avec de l'eau de corps réfringents contenant ladite somatotropine et d'autres protéines ; la dissolution desdits corps réfringents à un pH de 11,0 à 12,5 dans de l'urée environ 1,8 à 2,2 M pour que la concentration de la somatotropine soit d'environ 2 à 10 g/l ; le maintien de ladite solution audit pH pendant environ 2 à 20 minutes ; la dilution de ladite solution d'un facteur d'au moins 3 avec de l'eau ; l'ajustement du pH de ladite solution diluée entre 11,3 et 12,0 pour que la concentration de l'urée soit d'environ 0,4 molaire et que la concentration de la somatotropine soit d'environ 0,4 à 2,0 g/l ; et le maintien de ladite solution pendant 5 à 10 heures.

7. Procédé selon la revendication 6, dans lequel ladite somatotropine est de la somatotropine humaine, bovine, porcine, ovine, équine, caprine, aviaire ou de poisson.

8. Procédé selon la revendication 7, dans lequel ladite durée est de 7 à 8 heures.

**4M UREA PROCESS**

**AQUEOUS PROCESS**

**2M UREA PROCESS**

| | | |
|---|---|---|
| WASHED INCLUSION BODIES | WASHED INCLUSION BODIES | WASHED INCLUSION BODIES |
| DISSOLVE AT pH 12.0 4M UREA SOMATOTROPIN CONTENT 5 G/L HOLD 20 MINUTES | DISSOLVE AT pH 12.2 NO UREA SOMATOTROPIN CONTENT 1 G/L HOLD 20 MINUTES | DISSOLVE AT pH 12.0 2M UREA SOMATOTROPIN CONTENT 1 G/L HOLD 5 MINUTES |
| ADJUST TO pH 10.8 AGE 5 HOURS | ADJUST TO pH 11.5 AGE 10 HOURS | DILUTE FIVE-FOLD INTO pH 11.5 WATER READJUST TO pH 11.5 UREA NOW IS 0.4M TOTAL PROTEIN 2.4 G/L AGE 8 HOURS |

FIG. 1

FIG. 2

FIG. 3